# EUROPEAN PATENT APPLICATION

(11) **EP 2 546 343 A1**
(43) Date of publication of application: **16.01.2013**
(21) Application number: 11753496.6
(22) Date of filing: 11.03.2011
(51) Int. Cl.: C12N 15/09, C12N 1/15, C12N 1/19, C12N 1/21, C12N 5/10, C12N 9/02

(54) **NOVEL LIPOXYGENASES DERIVED FROM LEMNA PAUCICOSTATA**

(30) Priority: 11.03.2010 JP 2010055099
(71) Applicant: Shiseido Company, Ltd., Chuo-ku Tokyo 104-8010 (JP)
(72) Inventor: YOKOYAMA, Mineyuki, Yokohama-shi Kanagawa 236-8643 (JP); TAKAGI, Kazuteru, Yokohama-shi Kanagawa 236-8643 (JP); KAMICHI, Sari, Yokohama-shi Kanagawa 236-8643 (JP)
(74) Representative: Henkel, Breuer & Partner
(86) International application number: PCT/JP2011/055834
(87) International publication number: WO 2011/111838

(57) **Abstract**

Provided are novel, highly-active, 9'-product-specific lipoxygenase cDNAs derived from Lemna paucicostata strain SH and Lemna paucicostata strain 441. Also provided are proteins coded by said lipoxygenase cDNAs.

## Description

### [Technical Field]

The present invention relates to a novel highly active form of position 9 product specific lipoxygenase gene derived from Lemna paucicostata, a method of producing highly active form of position 9 product specific lipoxygenase by using the lipoxygenase gene, and an expression product of said lipoxygenase gene.

### [Background Art]

Lipoxygenase is an enzyme having an activity to directly introduce molecular oxygen into unsaturated fatty acid having 1,4-pentadiene structure, such as linoleic acid, linolenic acid (carbon number 18), or arachidonic acid (carbon number 20) to produce hydroperoxyl group (-OOH), and is known to be present in plants, animals, and eukaryotic microorganisms, such as fungis, yeast, and so on (Uchiyama Mitsuru, et al., lipid peroxide and biological body, 23 - 26, 1985, Japan Scientific Societies Press). In animals, since this enzyme relates to the process of producing prostaglandin from arachidonic acid, research is most ahead (Yamamoto Shouzo, Protein Nucleic acid Enzyme, 44, 1132-1138, 1999). Lipoxygenase in plants is known to be classified to an enzyme which shows specificity to two positions, position 9 and position 13, as using a unsaturated fatty acid with carbon number of 18, such as α-linolenic acid, linoleic acid, as a substrate (for example, Uchiyama Mitsuru, et al., lipid peroxide and biological body, 23 - 26, 1985, Japan Scientific Societies Press, and Grechkin et al., Eur. J. Biochem., 199, 451-457, 1991). Position 9 and position 13 specific lipoxygenase was known to be present in potato, soybean, tomato, pea, barley, wheat, corn, cucumber, and so on, and some cDNAs have been isolated. However, position 13 product specific lipoxygenase, which relates to a metabolic pathway of jasmonic acid, which attracts attention as a plant hormone, is mainly investigated, and position 9 product specific lipoxygenase is relatively less investigated. Position 13 product specific lipoxygenase derived from soybean is commercially available from Sigma (Sigma, Product Number L7395) as a test reagent, while position 9 product specific lipoxygense is not commercially available.

The present inventors have found a α-ketol unsaturated fatty acid (hereinafter referred to as KODA) having a structure represented by the following Formula: 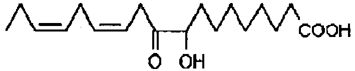 having a floral bud-formation promoting activity, a plant activation activity and a plant growth regulating activity comprising them, and KODA is known to be derived from α-linolenic acid by acting position 9 product specific lipoxygenase and allene oxide synthase (Japanese Unexamined Patent Publication (Kokai) No. 9-295908, Japanese Unexamined Patent Publication (Kokai) No. 11-29410, Japanese Unexamined Patent Publication (Kokai) No. 2001-131006, and Japanese Unexamined Patent Publication (Kokai) No. 2009-17829).

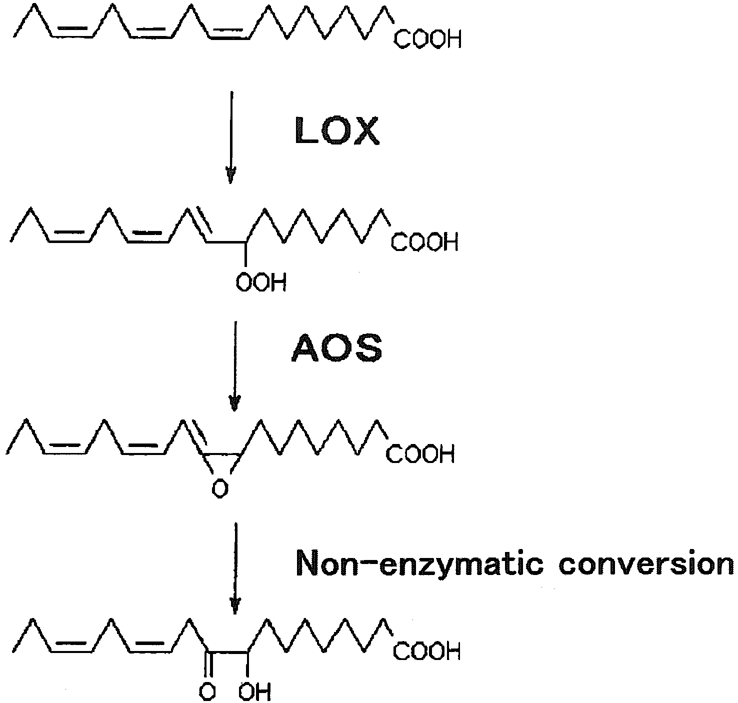

KODA was known to be present in a variety of plant species, and Lemna paucicostata that was subjected to stress was known to release KODA at a very high level (a few hundred-fold higher) compared to other plants ( Japanese Unexamined Patent Publication (Kokai) No. 11-29410). Thus, it was estimated that in Lemna paucicostata position 9 product specific lipoxygenase which has higher activity than in other plants are present. However, cDNA of position 9 product specific lipoxygenase has not been isolated, and the sequence thereof has been unknown.

When KODA is to be produced by enzymatic synthesis with α-linolenic acid or linoleic acid as the starting substance, position 9 product specific lipoxygenase is required. However, position 9 product specific lipoxygenase is not commercially available, and when cDNA of currently known position 9 product specific lipoxygenase was expressed in Escherichia coli, most of them are turned out to be insoluble, and thus it was difficult to obtain active protein at large quantities. In addition, even the extraction from a plant requires a lot of time and effort on obtaining and processing materials, and the activity of position 9 product specific lipoxygenases obtained to date was low. Therefore, there has been a need for establishing a technology to prepare highly active form of position 9-product specific lipoxygenase at large amount.

### [Citation List]

### [Patent Literature]

PLT1: Japanese Unexamined Patent Publication (Kokai) No. 9-295908
PLT2: Japanese Unexamined Patent Publication (Kokai) No. 11-29410
PLT3: Japanese Unexamined Patent Publication (Kokai) No. 2001-131006
PLT4: Japanese Unexamined Patent Publication (Kokai) No. 2009-17829

### [Non patent literature]

NPL1: Uchiyama Mitsuru, et al., lipid peroxide and biological body, 23 - 26, 1985, Japan Scientific Societies Press
NPL2: Yamamoto Shouzo, Protein Nucleic acid Enzyme, 44, 1132-1138, 1999
NPL3: Grechkin et al., Eur. J. Biochem., 199, 451-457, 1991

### [Summary of Invention]

### [Technical Problem]

The problem to be solved by the present invention is to identify highly active form of position 9 product specific lipoxygenase gene useful for the expression, to isolate cDNA, and to provide an expression product of said lipoxygenase gene, thereby to provide a method of preparing KODA in high yield by using the lipoxygenase.

### [Solution to Problem]

In intensive research to solve the above problem, and to isolate new lipoxygenase cDNA from Lemna paucicostata, the present inventors conducted a screening of various strains of Lemna paucicostata for the ability of producing KODA in order to obtain the cDNA of lipoxygenase having a higher activity. Specifically 62 strains of Lemna paucicostata were subjected to the screening, thereby it was surprisingly found that Lemna paucicostata SH strain has a KODA producing activity 10-fold or more higher than the mean of other Lemna paucicostata strains.

The present inventors have isolated lipoxygenase cDNA from Lemna paucicostata SH strain and Lemna paucicostata 441 strain, which is used as a representative strain, expressed the cDNA in Escherichia coli as host cell, and examined the product specificity. As a result, it was revealed that these lipoxygenases were confirmed to have position 9-product specific lipoxigenase activity, and that the activity thereof was higher than that of rice position 9 product specific lipoxigenase, which has been conventionally used.

Thus, the present inventors provide a novel position 9 product specific lipoxygenase gene derived from Lemna paucicostata and a protein encoded by said gene. Specifically, the present invention provides a gene consisting of a DNA having a base sequence selected from the group consisting of SEQ ID NOs: 1, 3 and 5, a gene consisting of a DNA substantially identical to said DNA, and proteins encoded by these genes. Furthermore, the present inventors provide an expression vector having the above DNA and a vector fragment bound to each other therein. Furthermore, the present invention also relates to a transformant of a microorganism, an animal cell or an insect cell transformed with said expression vector. The position 9 product specific lipoxigenase of the present invention can be obtained by culturing said transformant, and extracting and purifying the position 9 product specific lipoxigenase.

### [Advantageous Effects of Invention]

Position 9 product specific lipoxigenase of the present invention has a high activity compared to the conventionally used position 9 product specific lipoxigenase, and further maintains its high activity when expressed in Escherichia coli. Therefore, α-ketol unsaturated fatty acid such as KODA can be prepared efficiently through enzymatic synthesis which uses α-linolenic acid or linoleic acid, etc., as a starting material by using the position 9 product specific lipoxigenase.

### [Brief Description of Drawings]

[Fig. 1]

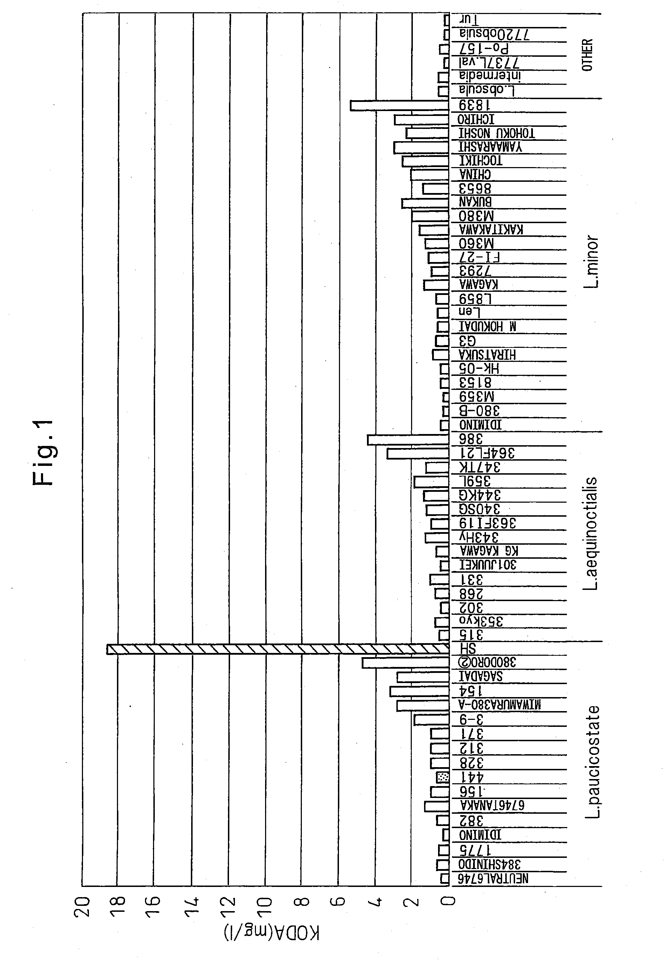

   Fig. 1 is a graph showing the amount of KODA produced in 62 strains of Lemna paucicostata.
[Fig. 2A]

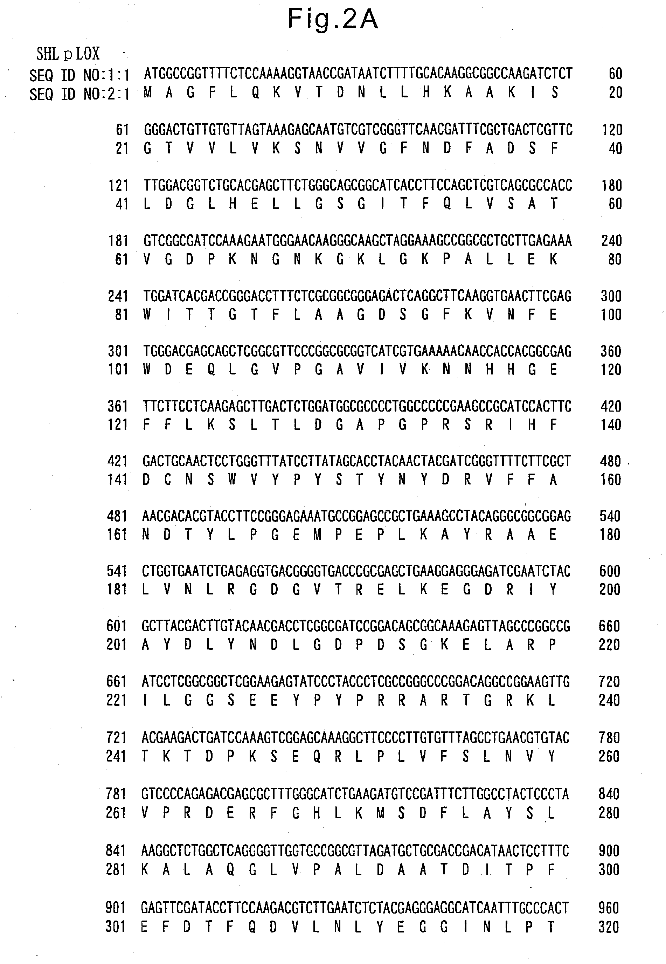

   Fig. 2A shows the sequence of the LOX gene of Lemna paucicostata SH strain.
[Fig. 2B]

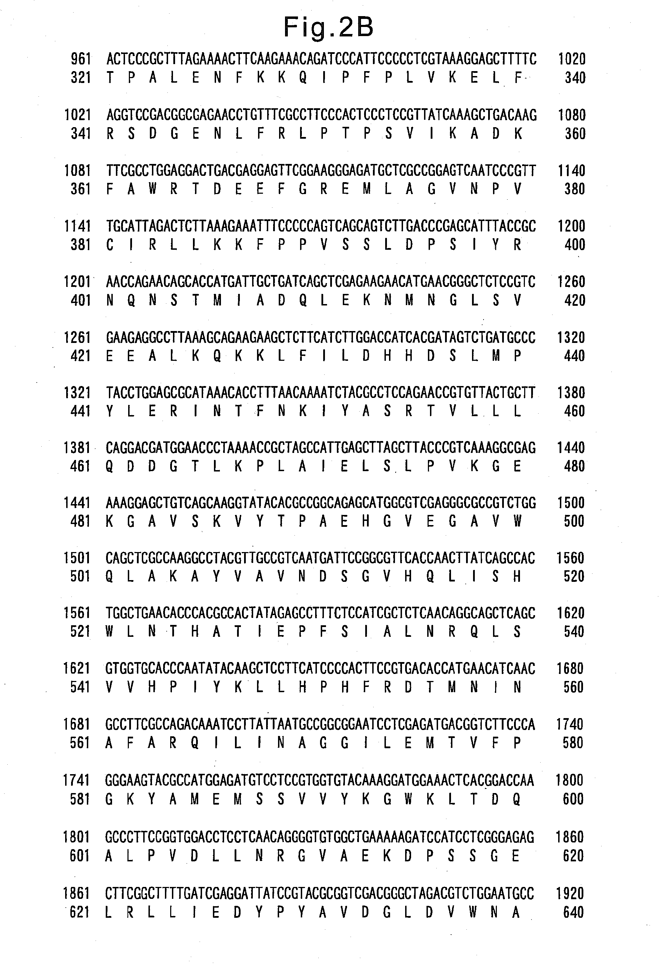

   Fig. 2B shows the continuation of the sequence of Fig. 2A.
[Fig. 2c]

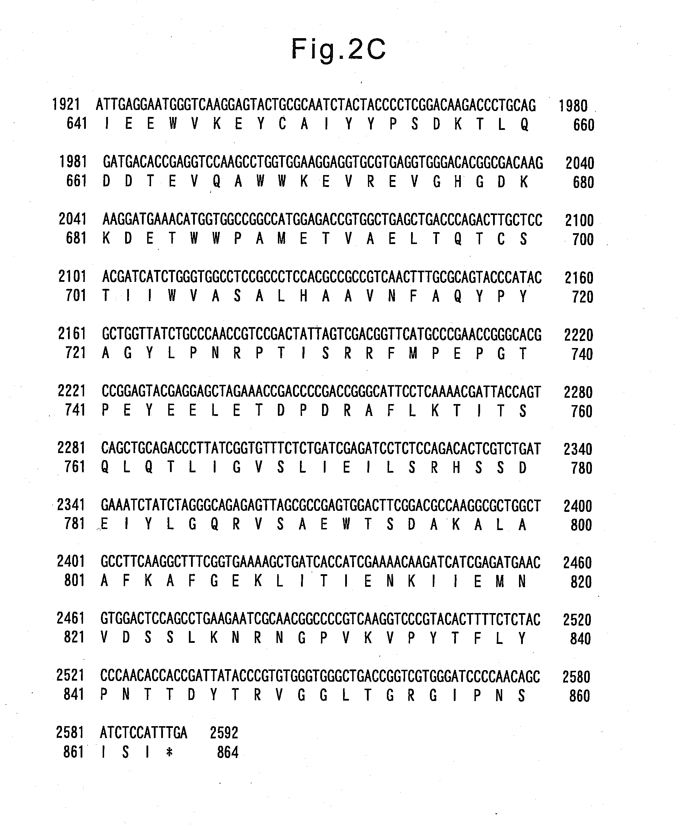

   Fig. 2C shows the continuation of the sequence of Fig. 2B.
[Fig. 3A]

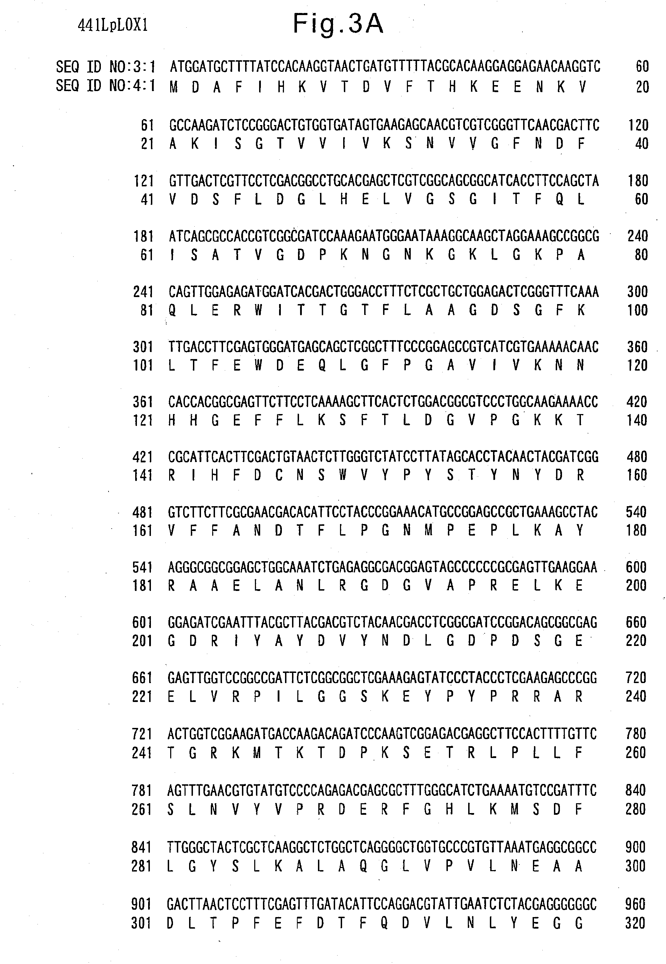

   Fig. 3A shows the sequence of LOX1 gene of Lemna paucicostata 441 strain.
[Fig. 3B]

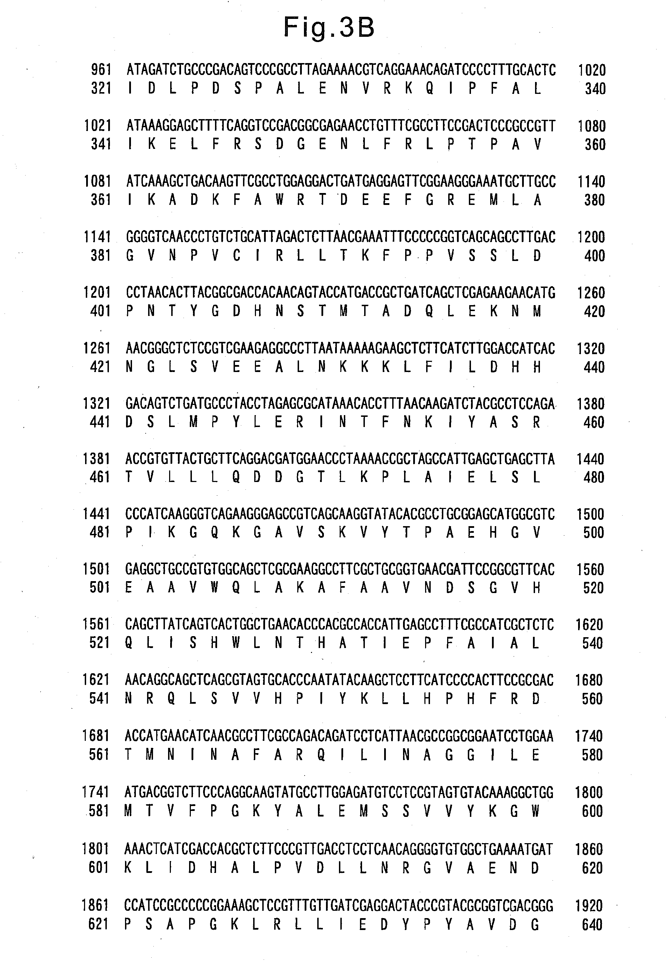

   Fig. 3B shows the continuation of the sequence of Fig. 3A.
[Fig. 3C]

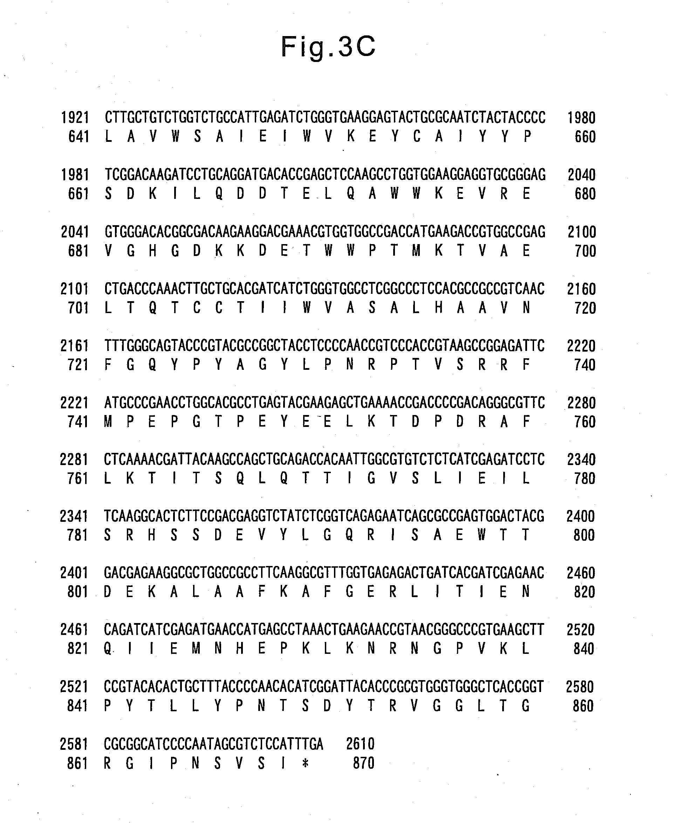

   Fig. 3C shows the continuation of the sequence of Fig. 3B.
[Fig. 4A]

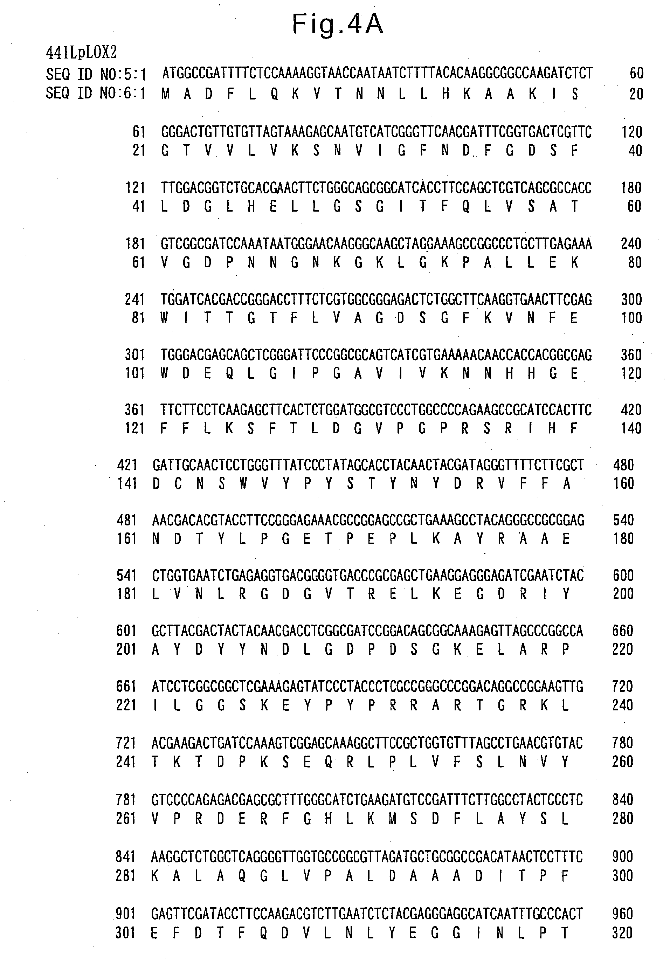

   Fig. 4A shows the sequence of LOX2 gene of Lemna paucicostata 441 strain.
[Fig. 4B]

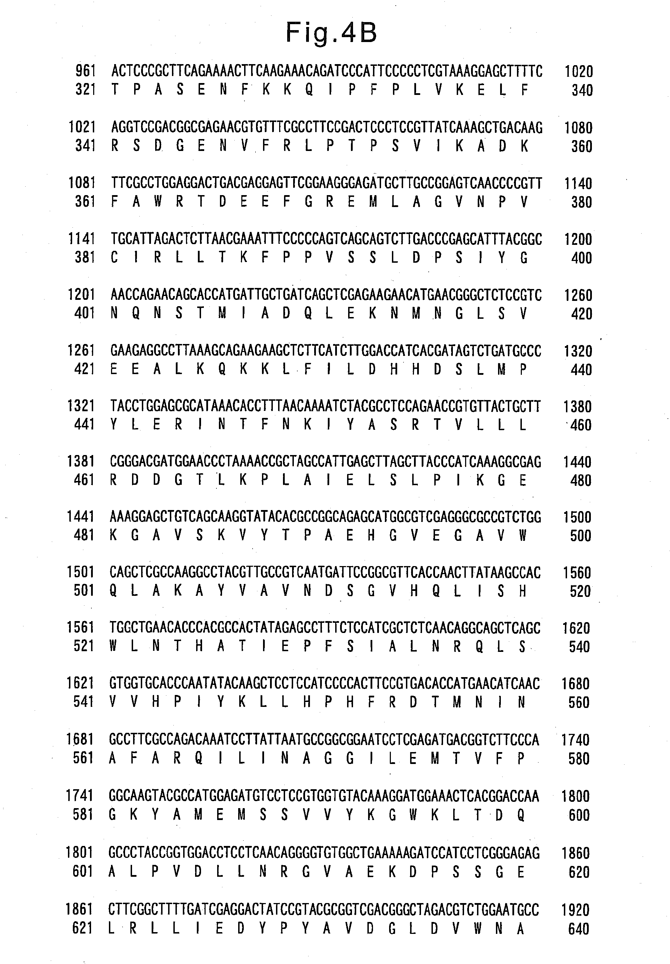

   Fig. 4B shows the continuation of the sequence of Fig. 4A.
[Fig. 4C]

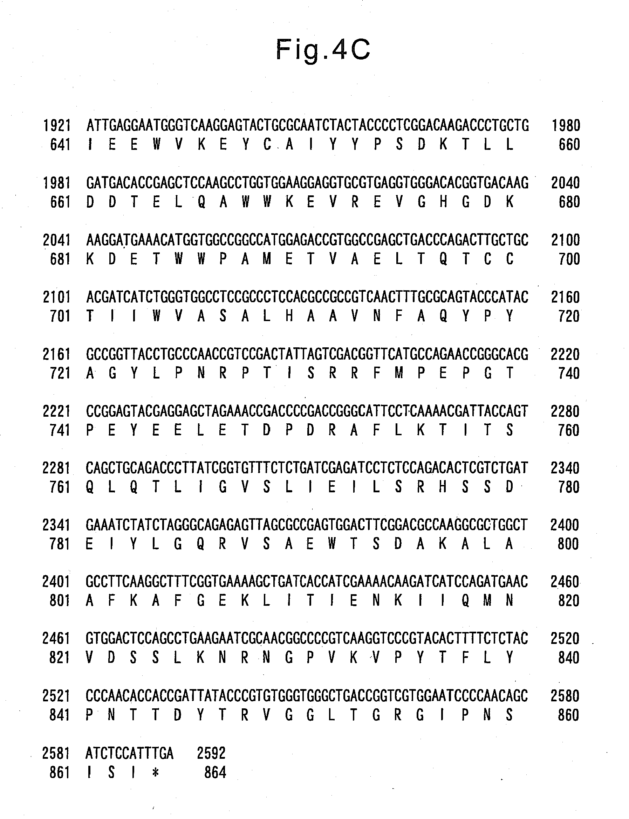

   Fig. 4C shows the continuation of the sequence of Fig. 4B
[Fig.5A]

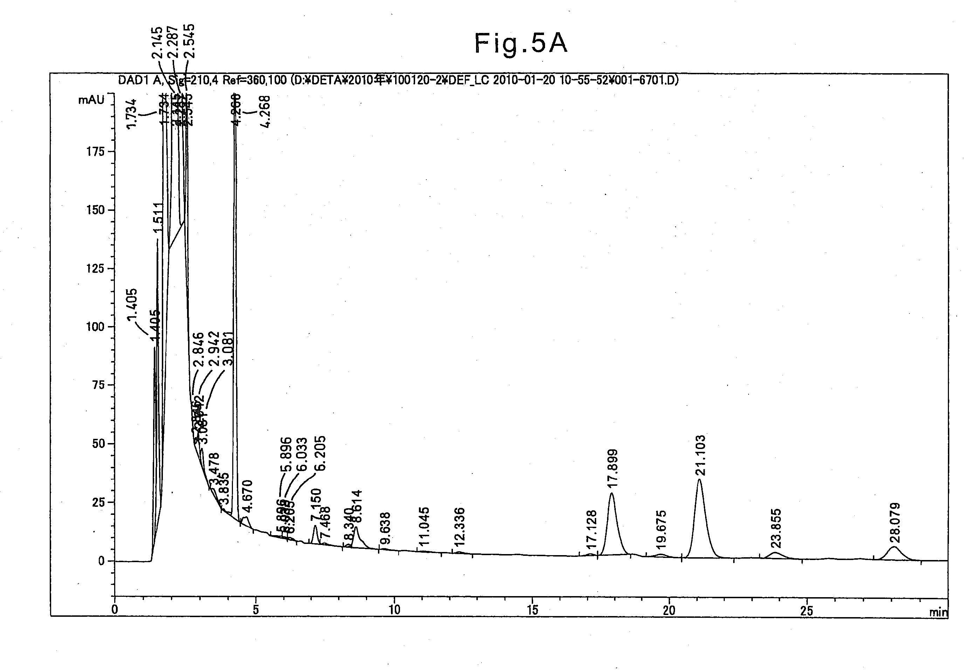

   Fig. 5A is a graph showing the HPLC chromatogram of linolenic acid-9-hydroperoxide synthesized by using r9-LOX1.
[Fig. 5B]

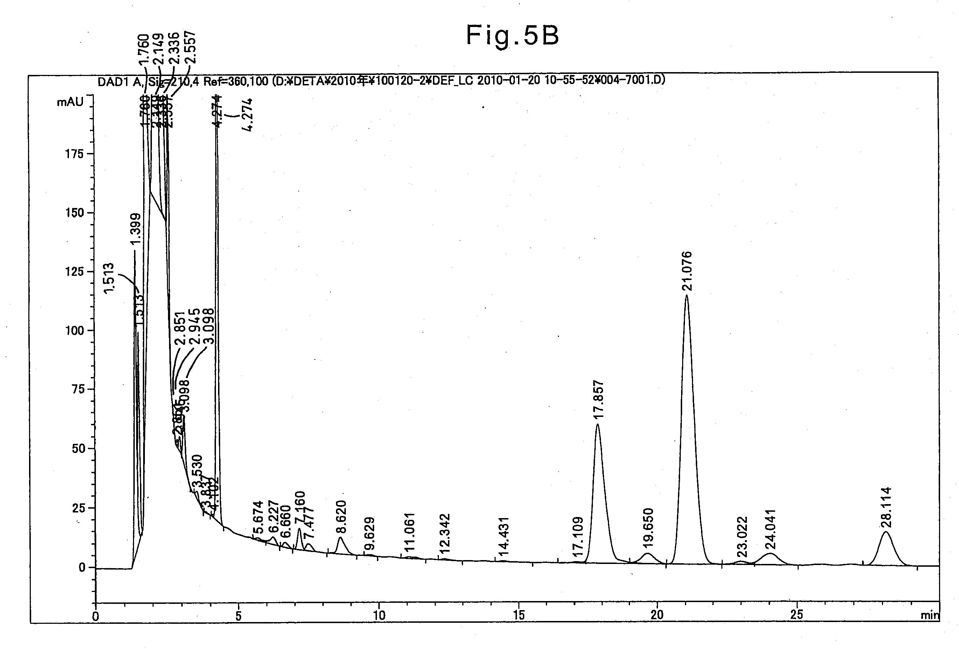

   Fig. 5B is a graph showing the HPLC chromatogram of linolenic acid-9-hydroperoxide synthesized by using SHLpLOX.
[Fig. 5C]

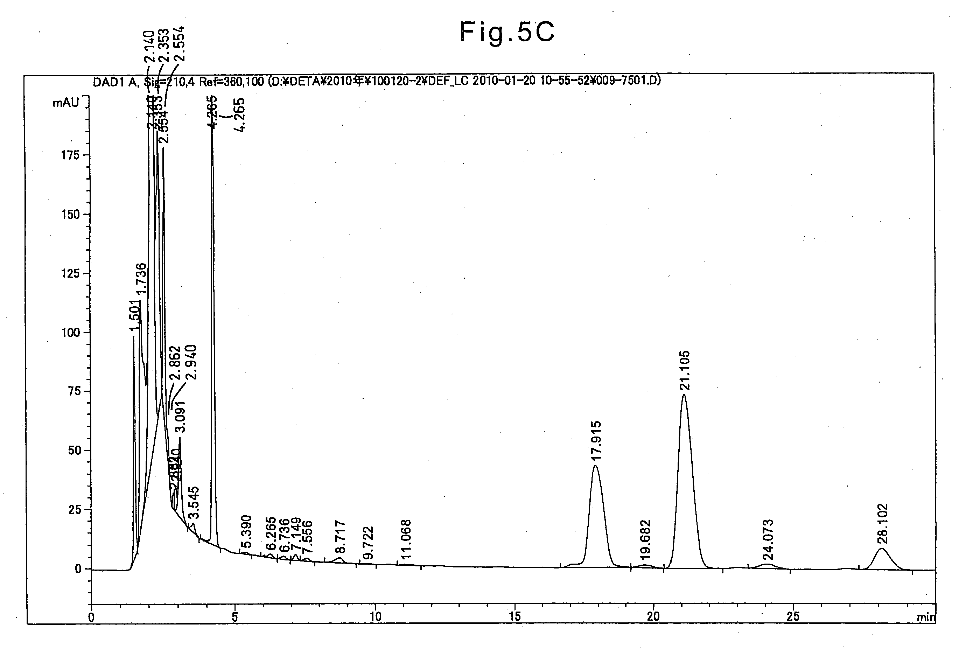

   Fig. 5C is a graph showing the HPLC chromatogram of linolenic acid-9-hydroperoxide synthesized by using 441LpLOX1.
[Fig. 5D]

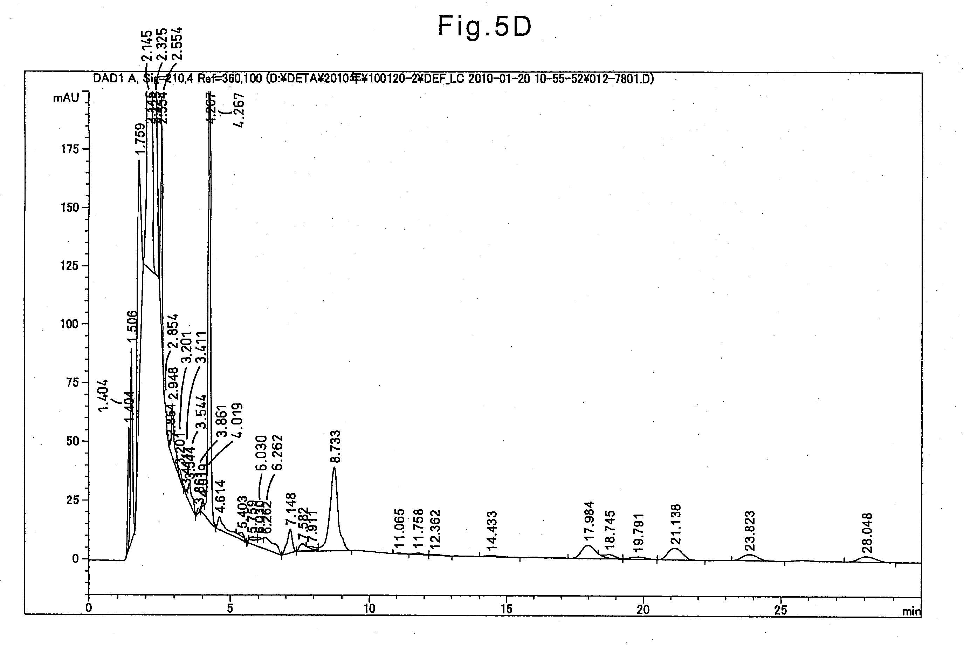

   Fig. 5D is a graph showing the HPLC chromatogram of linolenic acid-9-hydroperoxide synthesized by using 441LpLOX2.
[Fig. 6]

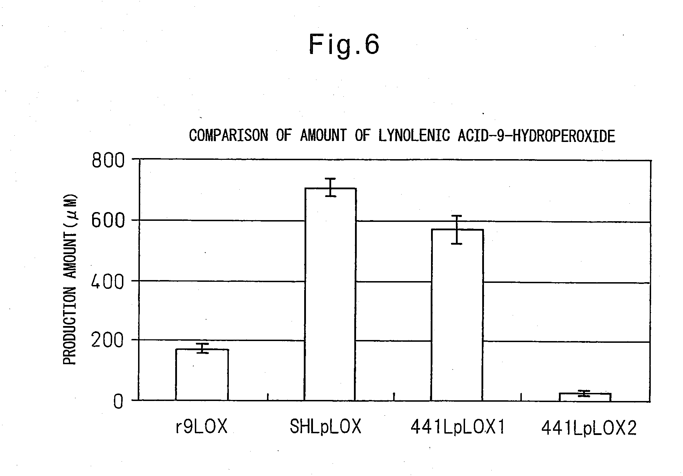

   Fig. 6 is a graph comparing the lypoxygenase activity of SHLpLOX, 441 LpLOX1, 441LpLOX2 and r9-LOX1, which is used as a control lypoxygenase.

### [Description of Embodiments]

The embodiments of the present invention are explained below. The present invention relates to a novel position 9 product specific lipoxygenase gene derived from Lemna paucicostata and a protein encoded by said gene. Specifically, the present invention relates to a gene consisting of a DNA having a base sequence selected from the group consisting of SEQ ID NO: 1, SEQ ID NO: 3 and SEQ ID NO: 5, and a DNA substantially identical to said DNA, and to a protein encoded by the gene. It is preferable that the base sequence is the sequence represented by SEQ ID NO: 1 or SEQ ID NO: 3. It is further preferable that the base sequence is the sequence represented by SEQ ID NO: 1. As used herein the term "substantially identical DNA" refers to a DNA that has at least 70% identity to the base sequence of the reference DNA. The identity may preferably be at least 80%, at least 90%, at least 95%, at least 97%, at least 98%, at least 99%, at least 99.5%, or at least 99.9%. Furthermore, "substantially identical DNA" also refers to a DNA that can hybridize under a high stringent condition to a DNA consisting of a base sequence complementary to the reference DNA. The "substantially identical DNA", when transcribed and translated, has the same enzyme activity as that of a protein encoded by the reference DNA, i.e., the position 9 product specific lipoxygenase activity. Furthermore, the present invention relates to a DNA that encodes a protein consisting of an amino acid sequence selected from the group consisting of SEQ ID NO: 2, SEQ ID No:4 and SEQ ID NO:6, and a DNA that encodes a protein consisting of an amino acid sequence substantially identical to said amino acid sequence. As used herein, "a protein consisting of substantially identical amino acid" is explained in detail below. In addition, the present invention also relates to a DNA fragment encoding a protein which has lypoxygenase activity.

Hybridization can be carried out by a known method or a method based on said method, such as a method described in J. Sambrook et al., Molecular Cloning, 2nd., Cold Spring Harbor Laboratory Press, 1989. In this connection, a high stringent hybridization condition refers to, for example, a NaCl concentration of about 10-40 mM, preferably about 20 mM, and a temperature of about 50-70°C, preferably about 60-65°C.

The present invention also relates to a protein consisting of an amino acid sequence selected from the group consisting of SEQ ID NO: 2, SEQ ID NO:4, and SEQ ID NO: 6, and a protein consisting of an amino acid sequence substantially identical to said amino acid sequence. It is preferable that the amino acid sequence is the sequence of SEQ ID NO:2 or 4. It is most preferable that the amino acid sequence is the sequence of SEQ ID No:2. As used herein, "a protein consisting of a sequence substantially identical to amino acid" refers to a protein that consists of an amino acid sequence in which one or a few amino acids have been deleted, substituted or added in the amino acid sequence of the reference protein, and that has the activity of the reference protein, i.e., position 9 product specific lypoxygenase activity. Furthermore, "a protein comprising a substantially identical amino acid sequence" refers to a protein that comprises a sequence having an identity of at least 70% with the reference amino acid sequence and that has an lypoxygenase activity. The identity may preferably be at least 80%, at least 90%, at least 95%, at least 97%, at least 98%, at least 99%, at least 99.5%, or at least 99.9%. In addition, the present invention relates to a protein which is encoded by a DNA of the base sequence selected from the group consisting of SEQ ID No:1, SEQ ID NO:3, and SEQ ID No:5, or a DNA substantially identical to said DNA, and which has position 9 product specific lypoxygenase activity. In addition, the present invention also relates to a protein fragment which has lypoxygenase activity.

As used herein SEQ ID NO: 1 represents the base sequence of the position 9 product specific lypoxygenase derived from Lemna paucicostata SH strain, and SEQ ID NO: 2 represents the amino acid sequence of said lypoxygenase. On the other hand SEQ ID No:3 represents the base sequence of the position 9 product specific lypoxygenase 1 derived from Lemna paucicostata 441 strain, and SEQ ID NO: 4 represents the amino acid sequence of said lypoxygenase, SEQ ID No:5 represents the base sequence of the position 9 product specific lypoxygenase2 derived from the Lemna paucicostata 441 strain, and SEQ ID NO:6 represents the amino acid sequence of said lypoxygenase. These concrete sequences are shown in Fig. 2A - 4C.

Position 9 product specific lypoxygenase activity means an enzymatic activity which uses an unsaturated fatty acid having carbon number of 18, such as linoleic acid as a substrate, and which introduces molecular oxygen as a hydroperoxide group into carbon at position 9 of the unsaturated fatty acid.

As used herein, position 9 product specific lypoxygenase or a protein having position 9 product specific lypoxygenase activity refers to one that is recognized to have a significant position 9 product specific lypoxygenase activity when determined by the activity determination method described below. For example, compared to the activity of position 9 product specific lypoxygenase having an amino acid sequence of SEQ ID NO: 2 or SEQ ID No:4, those having an activity of at least 10%, at least 50%, at least 70%, at least 80%, at least 90%, more preferably at least 95%, and furthermore 100% or more are encompassed.

The gene (cDNA) of the present invention can be obtained by, for example, the following procedure. From an amino acid sequence common to a plant-derived lypoxygenase, primers are designed, and RT-PCR is carried out to obtain a cDNA fragment. This fragment is labelled, as needed, with ³²P or digoxgenine, etc., and it is used in 5'-RACE or 3'-RACE, or screening is carried out in accordance with a common method by using cDNA library constructed in a standard method, thereby the full-length of lypoxygenase gene (cDNA) can be obtained. The determination of the base sequence can be conducted by a common method, such as the Sanger method and the Maxam Gilbert method.

In an alternative method, Lemna paucicostata-derived position 9 product specific lypoxygenase is purified using ammonium sulfate fractionation or various chromatographic methods, separated by SDS polyacrylamide gel electrophoresis, electrically transferred to a PVDF membrane etc., and excised a band detected with a dye such as Coomassie brilliant blue. Then, the N-terminal amino acid sequence is determined with a protein sequencer. Based on this sequence, primers are designed, and PCR is carried out according to the method mentioned above to obtain the full-length of the lypoxygenase gene gene (cDNA).

The isolated gene (cDNA) can be inserted into a plasmid, virus etc., by a commonly known method so as to prepare an expression vector, which is then introduced into the host cells such as a microorganism, or cultured cells of animal cells, plant cells, insect cells, etc. Then, the cells are allowed to express the gene, thereby the protein can be produced in large quantities. Furthermore, as the protein-expression system, a cell-free protein expression system, for example a system using a wheat germ extract, may be used.

As a vector, an Escherichia coli-derived plasmid such as pBR322, pBR325, pUC18 and pUC119, a Bacillus subtilis-derived plasmid such as pUB110, pTP5 and pC194, a yeast-derived plasmid such as pSH19 and pSH15, a bacteriophage such as λ phage, an animal virus such as retrovirus, vaccinia virus and baculovirus, and pA1-11, pXT1, pRC/CMV, pRC/RSV, pcDNAI/Neo etc., can be used. pET23d (Novagen), which is a commercially available vector, can be used in the present invention.

The expression vector comprising the gene of the present invention can be produced by ligating the gene of the present invention downstream to a promoter of a suitable vector via a suitable restriction site by a known method. The promoter to be used in the present invention may not be specifically limited as long as it is a suitable promoter corresponding to the host to be used for the expression of the gene. For example, when an animal cell is used as the host, SRα promoter, SV40 promoter, LTR promoter, CMV promoter, HSV-TK promoter, β-actin promoter etc. can be mentioned. When the host is a bacteria of the genus Escherichia such as Escherichia coli, trp promoter, lac promoter, recA promoter, λPL promoter, lpp promoter, T7 promoter etc. may preferably be used, when the host is a bacteria of the genus bacillus, SPO1 promoter, SPO2 promoter, pen promoter may preferably be used, and when the host is a yeast, PHO5 promoter, PGK promoter, GAP promoter, ADH promoter etc., may preferably be used. When the host is an insect, polyhedron promoter, P10 promoter etc., may be preferred. As desired, the expression vector may further contain an enhancer, a splicing signal, a polyA addition signal, a selection marker, SV40 replication origin, etc.

The host cell may be, but not limited to, a procaryotic cell or an eucaryotic cell that is conventionally used as the host for producing recombinant protein, and may include a mammal cell, an insect cell, a plant cell or a fungal cell, and preferably microorganisms such as bacteria or fungi. The term "fungus" is intended to encompass filamentous microorganisms and yeasts. Examples of suitable bacteria include Gram positive bacteria including genus Escherichia such as Escherichia coli, genus Bacillus such as Bacillus subtilis, and genus Streptomyces such as S. lividans, and Gram negative bacteria including genus Pseudomonas such as P. cepacia. The cells may be of fungi, i.e. yeast or filamentous cells. Yeast may be cells of genus Saccharomyces such as S. cereviceae. Filamentous host orgamisms may be, for example, Aspergillus strains such as A. niger. As insect cells, Sf cells, MG1 cells, BmN cells etc. may be mentioned. As animal cells, for example, monkey cell COS-7 cells, Vero cells, Chinese hamster cells CHO etc. may be mentioned. As used herein, Escherichia coli cells may specifically be preferred.

For the transformation of host cells, a known method such as the calcium chloride method, the calcium phosphate coprecipitation method, the DEAE dextran method, the lipofectin method, the protoplast polyethylene fusion method, the electroporation method etc. can be used, and may be selected as appropriate depending on the host cell to be used.

The protein of interest can be recovered by culturing the recombinant host cells obtained in a suitable culture medium, separating the cells from the culture liquid by a common procedure including, but not limited to, centrifugation or filtration, disrupting the cells as needed, precipitating the proteinaceous components of the supernatant or the filtrate by a salt such as ammonium sulfate, and then performing various chromatographic procedures such as ion exchange chromatography and an affinity chromatography.

If desirable, by ligating a suitable signal sequence for secretion in microorganisms or animals to upstream of a DNA encoding the protein of the present invention, said protein can be secreted and expressed in the culture medium. Such a DNA modified to a secretary type is useful since it facilitates the purification of the protein secreted into the culture medium. As examples of such a signal sequence, there can be mentioned the pel B signal (S.P. Lei et al., J. Bacteriology, 169: 4379-4383, 1987) in the case of Escherichia coli, α factor signal (A.J. Brake, Yeast Genetic Engineering, p269 Butterworth, 1989) in the case of yeast, the signal SG-1 of immunoglobulin (H. Maeda et al., Hum. Antibod. Hybridomas, 2: 124-134, 1991), C25 signal (WO94/20632) etc. in the case of animal cells.

The lypoxygenase activity can be determined by the method generally used. For example, position 9 lypoxygenase is acted with a substrate, such as α-linolenic acid or linoleic acid, and then a method for detecting an increase of absorbance at 234nm, which is resulted from the production of an enzymatic reaction production, hydroxyperoxide form, for detecting reduction of dissolved oxigen in reaction solution, which is resulted from the consumption of molecular oxygen, or directly detecting hydroxyperoxide form in HPLC can be used. In addition, the position 9-product specificity can be detected by using normal-phase HPLC, etc., with a silica gel column as described in a prior document (Yamamoto et al., Lipids, 15, 1-5, 1980).

The protein of the present invention may be useful as a reagent for use in the synthesis of various linoleic acid and linolenic acid derivatives. Specifically, the protein of the present invention is useful as an enzyme to be used when an α-ketol unsaturated fatty acid, specifically KODA, useful as a plant growth regulating agent, is enzymatically synthesized from linoleic acid or linolenic acid. Also, it may be estimated that by introducing the gene of the present invention into a plant, the metabolism of linoleic acid or linolenic acid present in plant by position 9 product specific lypoxygenase can be controlled.

The present invention will now be explained more specifically with reference to examples below. However, these examples do not limit the technical scope of the present invention in any way.

### Examples

### Example 1: Screening of a high KODA-producing Lemna paucicostata strain

62 types of Lemna paucicostata harvested from different places were prepared and were passaged in the 1/2-diluted Hutner's medium under continuous illumination from a 24-25°C daylight fluorescent light. The 1/2-diluted Hutner's medium had the following ingredients:

| | |
|---|---|
| Sucrose | 10 g/l |
| K₂HPO₄ | 200 mg/l |
| NH₄NO₃ | 100 mg/l |
| EDTA free acid | 250 mg/l |
| Ca(NO₃)·4H₂O | 176 mg/l |
| MgSO₄·7H₂O | 250 mg/l |
| FeSO₄·7H₂O | 12.4 mg/l |
| MnCl₂·4H₂O | 8.92 mg/l |
| ZnSO₄·7H₂O | 32.8 mg/l |
| Na₂MoO₄·2H₂O | 12.6 mg/l |
| H₃BO₃ | 7.1 mg/l |
| Co(NO₃)·6H₂O | 0.1 mg/l |
| CuSO₄·5H₂O | 1.97 mg/l |

and pH was adjusted to 6.2-6.5 with KOH (50%).

The grown Lemna paucicostata was unfolded on a filter paper, and after allowing to stand for 2 hours, it was immersed in water for 1 hour. For the water, the concentration of KODA was analyzed using high performance liquid chromatography (HPLC; column: TYPE UG120 5 µm, SIZE: 4.6 mm I.D.×250 mm; guard filter: INERTSTL 4.6mm×50 mm; eluent: 50% acetonitrile+0.1% trifluoroacetic acid; condition: absorption wavelength 210 λ(nm), flow rate: 1 ml/min, column temperature: 40°C). The mean of the amount produced of KODA in all Lemna paucicostata strains was 4.97 µM. Among them, Lemna paucicostata SH strain produced 60.2 µM of KODA, which is about 12-times as much as the mean amount of KODA produced, giving a very high production amount (Fig. 1).

### Example 2: Cloning of lipoxygenase derived from Lemna paucicostata SH strain and measurement of the activity thereof

From Lemna paucicostata SH strain and Lemna paucicostata 441 strain, total RNA was extracted by using the RNeasy Plant Mini Kit (QIAGEN), and then cDNA was synthesized by using 1.8 µg of total RNA of each strain of Lemna paucicostata as the template in LongRange 2 Step RT-PCR Kit (QIAGEN).
Then, degenerate PCR (PCR condition: initial denaturation at 94°C for 3 minutes; a cycle comprising 94°C for 0.5 minute, 47°C for 0.5 minute, and 72°C for 1.3 minute is carried out for 39 times) was carried out by using the cDNA as the template, and using the following degenerate primers (LpDPf, LpDPr) to obtain a partial sequence of 9-lipoxygenase of interest.
LpDPf: 5'-GCITGGMGIAGIGAYGARGARTTY-3' (SEQ ID NO: 7)
LpDPr: 5'-GCRTAIGGRTAYTGICCRAARTT-3' (SEQ ID NO: 8)
wherein, I represents inosine.
In order to design primers for cloning Lemna paucicostata lypoxygenase gene (cDNA), these degenerate primers were constructed from a region wherein high homology is present in amino acid sequences of lypoxygenase derived from plants which had been cloned already (soybean, potato, tomato, barley, rice).

One sequence of product and two sequences of products having about 1.0kb, which are respectively corresponds to the sentral region of lypoxygenase cDNA from 441 strain are obtained, are obtained, and determined for the base sequences. Based on the obtained sequence information, BLAST search was carried. As a result, these three sequences exhibited a high homology with LOX derived from a plurality of known plants (75% with Corylus avellana, 74% with Actinidia deliciosa, 75% with Solanum tuberosum, 76% with Oryza sativa, 74% with Nicotiana tabacum, 75% with Cucumis sativus, 73% with Arabidopsis thaliana, etc.).

Based on this sequence information, primers for the following 3' or 5' RACE method (Rapid Amplification of cDNA end) were constructed:
3'-Race primer
   SH-3'-TP: 5'-AGCTCTTCATCTTGGACC-3' (SEQ ID NO: 9)
   441-1-3'-TP: 5'-AAGCTTCTTCATCCCCACTTCC-3' (SEQ ID NO:10)
   441-2-3'-TP: 5'-AAGCTCCTTCATCCCCACTTCC-3' (SEQ ID No:11)
5'-Race primer
   SH-5'-TP: 5'-TTTCATCCTTCTTGTCGC-3' (SEQ ID NO: 12)
   441-1-5'-TP: 5'-GCTTGTATATTGGGTGCAC-3' (SEQ ID NO: 13)
   441-2-5'-TP: 5'-AAGCACTAACACGGTTCTGGAGG-3' (SEQ ID NO: 14)

### 3'-RACE

2 µg of total RNA obtained from SH strain or 441 strain was used as a template so as to synthesize cDNA by using Cap Fishing™ target full-length cDNA cloning Kit (Seegene). This cDNA is used as a template solution, and then PCR is each carried out by using adapter primer (3'-RACE primer) contained in a kit and SH-3'-TP, 441-1-3'-TP, or 441-2-3'-TP.

### 5'-RACE

2 µg of total RNA obtained from SH strain or 441 strain was used as a template so as to synthesize cDNA by using Cap Fishing™ target full-length cDNA cloning Kit (Seegene). This cDNA is used as a template solution, and then PCR is each carried out by using adapter primer (5'-RACE primer) contained in a kit and SH-5'-TP, 441-1-5'-TP, or 441-5'-TP.

The sequence of each product was determined, and then the products were connected into one sequence by using an appropriate restriction site so as to obtain whole length cDNA, which is then sub-cloned into pTAC-2 vector (BioDynamics).
Lypoxygenase obtained from Lemna paucicostata SH strain is named as SHLpLOX, and Lypoxygenase obtained from Lemna paucicostata 441 strain is named 441LpLOX1 and 441LpLOX2.

Regarding three kinds of novel LOXcDNAs derived from Lemna paucicostata 441 strain and SH strain, a DNA fragment comprising full length of translation region consisting of starting codon to terminate codon was obtained by using PCR with primers having NcoI clevage domain at 5' end, and NotI cleavage domain at 3' end, and then inserted into NcoI/NotI sites of commercially available expression vector, pET23d(Novagen), thereby plasmide pET23d/SHLpLOX, pET23d/441LpLOX1 and pET23d/441LpLOX2 were obtained. These plasmide was transformed into Escherichia coli BL21 (DE3) strain (Novagen). Then, the microorganism was inoculated into LB medium containing 10mg/L ampicilin, and then shake-cultured at 37 for overnight. 100µl of the culture medium was added to 10 ml of LB medium containing 10mg/L ampicilin, and then shake-cultured at 37 °C for 5 hours. the fungus body was recovered by centrifuging from the culture medium, and then transferred to TB medium containing 0.1mM isopropyl-β-D-thiogalactopyranocid (Wako), 100mg/l of ampicilin and cultured for 40 hours at 15 °C so as to induce the expression of lypoxygenase. After the culture, the fungus body was collected by centrifugation (wet weight 0.4g), and was dissolved in 10ml of BugBuster™ (Novagen). Then centrifugation (9000rpm, 5 minutes, 4°C) was carried out so as to collect supernatant (10ml) as a enzyme solution.

As a control enzyme solution of position 9 specific lypoxygenase, an enzyme solution obtained by expressing and extracting lypoxygenase as described above from Escherichia coli BL21 (DE3) transformed by a plasmid, wherein r9-LOX1, which is position 9 specific lypoxygenase derived from rice germ, was inserted into NcoI/NotI site of pET23d, was used.

The activity of lypoxygenase in the enzyme solutions of SHLpLOX, 441LpLOX1, 441LpLOX2 and r9-LpLOX1 which was used as a control, was determined. 25µ1 of 5mM α-lynolenic acid solution (including 0.05% Tween80), 10µl of 0.2M phosphate Na Buffer (pH7.0), 5µl of distilled water were added to 1.5ml of tube, and then 10µl of the enzyme solution was added to the tube, and allowed to react for 30 minutes. After the reaction was over, reactant was subjected to HPLC (reverse phase; Column: Capsule pack C-18 UG120 (4.6x250mm, Shiseido), Column temperature: 40°C, Mobile phase: 50% acetnitril (0.02%TFA), Flow rate 1 ml/min, Detection: 210nm) so as to detect the site specificity of produced lynolenic acid hydroperoxide and the production amount. The result of HPLC was shown in Figure 5A-D. As shown in a HPLC chart, a peak of linolenic acid-9-hydroxyperoxide (21.1 min) was confirmed in each chart of SHLp-LOX, 441LpLOX1, 441pLOX2, which revealed that the enzymes have position 9 product specific lypoxygenase activity.

The amount of lynolenic acid-9-hydroperoxide form was compared based on the results of HPLC. The result of the comparison was shown in Figure 6. The Novel LOX derived from Lemna paucicostata SH strain (SHLpLOX) and the novel LOX derived from Lemna paucicostata 441 strain (441LpLOX1) exert far higher activity of position 9-product specific lypoxygenase than that of r9-LOX1, which has been known to have strongest activity among the known position 9 -product specific lyopxygenase.

Among the novel LOXs derived from Lemna paucicostata, SHLpLOX that had the highest production amount of linolenic acid-9-hydroperoxide was subjected to kinetic analysis. Using a reaction mixture comprising 40 mM phosphate buffer (pH 6.0) and 0.1% Tween 80, the analysis was carried out at a temperature of 25°C. The substrate α-linolenic acid was tested at the substrate concentration of 10-100 µM. 100 µl of the reaction mixture was added into a cuvette, and absorbance at 234 nm was scanned over time for 10 minutes at an interval of 15 seconds using the SmartSpec Plus Spectrophotometer (Bio-Rad). The amount of the reaction product was calculated from the A₂₃₄ determined (e=25,000). Kinetic parameters were determined using the Hanes-Woolf plot ([S]/v versus [S] plot). As shown in Table 1, the result shows that the Kₘ value which is an affinity parameter for substrate is lower in SHLpLOX than in r9-LOX1, thereby indicating that SHLpLOX had a high affinity for the substrate α-linolenic acid. The maximum reaction velocity Vₘₐₓ is almost comparable between r9-LOX and SHLpLOX, while the k_{cat} value which is the number of reactions per unit time was higher in SHLpLOX than in r9-LOX1. The k_{cat}/Kₘ value which is an index of enzyme activity was about 1.6-fold higher in SHLpLOX than in r9-LOX1. This revealed that the novel 9-LOX derived from Lemna paucicostata SH strain is a very highly active 9-LOX.

**[Table 1]**

| Kinetic parameters of SHLpLOX and r9-LOX1 | | | | |
|---|---|---|---|---|
| | Kₘ (µM) | Vₘₐₓ (µmol min⁻¹) | k_{cat} (min⁻¹) | K_{cat}/Kₘ (×10⁵M⁻¹min⁻¹) |
| LpLOX-SH | 19.2±4.1 | 3.0±0.1 | 768.2±32.3 | 41.4±10.0 |
| r9-LOX1 | 22.9±1.6 | 2.8±0.6 | 614.1±124.6 | 26.7±4.2 |

## Claims

1. A gene consisting of a DNA selected from the group consisting of the following (a) to (e):
(a) a DNA consisting of a base sequence of SEQ ID NO: 1;
(b) a DNA that encodes a protein consisting of an amino acid sequence of SEQ ID NO: 2;
(c) a DNA encoding a protein that consists of an amino acid sequence having at least 99% identity to the amino acid sequence of SEQ ID No:2, and that has position 9 product specific lypoxygenase activity;
(d) a DNA encoding a protein that consists of an amino acid sequence in which one or a few amino acids have been deleted, substituted or added in the amino acid sequence of SEQ ID NO: 2, and that has position 9 product specific lypoxygenase activity;
(e) a DNA that hybridizes under a high stringent condition to a DNA comprising a base sequence complementary to a DNA comprising a base sequence represented by SEQ ID NO: 1, and that encodes a protein having position 9 product specific lypoxygenase activity.

2. An expression vector comprising the DNA according to claim 1 and a vector fragment bound to each other therein.

3. A transformant wherein the expression vector according to claim 2 has been introduced into the host microorganism, animal cell or plant cell.

4. A method of producing position 9 product specific lypoxygenase, **characterized in that** comprising culturing the transformant according to claim 3.

5. A protein selected from the group consisting of the following (a) to (d):
(a) a protein consisting of an amino acid sequence represented by SEQ ID NO: 2;
(b) a protein that consists of an amino acid sequence in which one or a few amino acids have been deleted, substituted or added in an amino acid sequence represented by SEQ ID NO: 2, and that has position 9 product specific lypoxygenase activity;
(c) a protein that consists of an amino acid sequence having an identity of at least 99% with amino acid sequence of SEQ ID NO:2, and has position 9 product specific lypoxygenase activity; and
(d) a protein that is encoded by a polynucleotide capable of hybridizing under a high stringent condition to a DNA comprising a base sequence complementary to a base sequence represented by SEQ ID NO: 1 and that has an position 9 product specific lypoxygenase activity.


SEQUENCE LISTING

<110>  Shiseido Company, Ltd.

<120>  Novel Lypoxygenase derived from Lemna paucicostata

<130>  Z568-PCT

<150>  JP2010-055099
<151>  2010-03-11

<160>  14

<170>  PatentIn version 3.1

<210>  1
<211>  2592
<212>  DNA
<213>  Lemna paucicostata

<400>  1
atggccggtt ttctccaaaa ggtaaccgat aatcttttgc acaaggcggc caagatctct      60

gggactgttg tgttagtaaa gagcaatgtc gtcgggttca acgatttcgc tgactcgttc     120

ttggacggtc tgcacgagct tctgggcagc ggcatcacct tccagctcgt cagcgccacc     180

gtcggcgatc caaagaatgg gaacaagggc aagctaggaa agccggcgct gcttgagaaa     240

tggatcacga ccgggacctt tctcgcggcg ggagactcag gcttcaaggt gaacttcgag     300

tgggacgagc agctcggcgt tcccggcgcg gtcatcgtga aaaacaacca ccacggcgag     360

ttcttcctca agagcttgac tctggatggc gcccctggcc cccgaagccg catccacttc     420

gactgcaact cctgggttta tccttatagc acctacaact acgatcgggt tttcttcgct     480

aacgacacgt accttccggg agaaatgccg gagccgctga aagcctacag ggcggcggag     540

ctggtgaatc tgagaggtga cggggtgacc cgcgagctga aggagggaga tcgaatctac     600

gcttacgact tgtacaacga cctcggcgat ccggacagcg gcaaagagtt agcccggccg     660

atcctcggcg gctcggaaga gtatccctac cctcgccggg cccggacagg ccggaagttg     720

acgaagactg atccaaagtc ggagcaaagg cttccccttg tgtttagcct gaacgtgtac     780

gtccccagag acgagcgctt tgggcatctg aagatgtccg atttcttggc ctactcccta     840

aaggctctgg ctcaggggtt ggtgccggcg ttagatgctg cgaccgacat aactcctttc     900

gagttcgata ccttccaaga cgtcttgaat ctctacgagg gaggcatcaa tttgcccact     960

actcccgctt tagaaaactt caagaaacag atcccattcc ccctcgtaaa ggagcttttc    1020

aggtccgacg gcgagaacct gtttcgcctt cccactccct ccgttatcaa agctgacaag    1080

ttcgcctgga ggactgacga ggagttcgga agggagatgc tcgccggagt caatcccgtt    1140

tgcattagac tcttaaagaa atttccccca gtcagcagtc ttgacccgag catttaccgc    1200

aaccagaaca gcaccatgat tgctgatcag ctcgagaaga acatgaacgg gctctccgtc    1260

gaagaggcct taaagcagaa gaagctcttc atcttggacc atcacgatag tctgatgccc    1320

tacctggagc gcataaacac ctttaacaaa atctacgcct ccagaaccgt gttactgctt 1380

caggacgatg gaaccctaaa accgctagcc attgagctta gcttacccgt caaaggcgag 1440

aaaggagctg tcagcaaggt atacacgccg gcagagcatg gcgtcgaggg cgccgtctgg 1500

cagctcgcca aggcctacgt tgccgtcaat gattccggcg ttcaccaact tatcagccac 1560

tggctgaaca cccacgccac tatagagcct ttctccatcg ctctcaacag gcagctcagc 1620

gtggtgcacc caatatacaa gctccttcat ccccacttcc gtgacaccat gaacatcaac 1680

gccttcgcca gacaaatcct tattaatgcc ggcggaatcc tcgagatgac ggtcttccca 1740

gggaagtacg ccatggagat gtcctccgtg gtgtacaaag gatggaaact cacggaccaa 1800

gcccttccgg tggacctcct caacaggggt gtggctgaaa aagatccatc ctcgggagag 1860

cttcggcttt tgatcgagga ttatccgtac gcggtcgacg ggctagacgt ctggaatgcc 1920

attgaggaat gggtcaagga gtactgcgca atctactacc cctcggacaa gaccctgcag 1980

gatgacaccg aggtccaagc ctggtggaag gaggtgcgtg aggtgggaca cggcgacaag 2040

aaggatgaaa catggtggcc ggccatggag accgtggctg agctgaccca gacttgctcc 2100

acgatcatct gggtggcctc cgccctccac gccgccgtca actttgcgca gtacccatac 2160

gctggttatc tgcccaaccg tccgactatt agtcgacggt tcatgcccga accgggcacg 2220

ccggagtacg aggagctaga aaccgacccc gaccgggcat tcctcaaaac gattaccagt 2280

cagctgcaga cccttatcgg tgtttctctg atcgagatcc tctccagaca ctcgtctgat 2340

gaaatctatc tagggcagag agttagcgcc gagtggactt cggacgccaa ggcgctggct 2400

gccttcaagg ctttcggtga aaagctgatc accatcgaaa acaagatcat cgagatgaac 2460

gtggactcca gcctgaagaa tcgcaacggc cccgtcaagg tcccgtacac ttttctctac 2520

cccaacacca ccgattatac ccgtgtgggt gggctgaccg gtcgtgggat ccccaacagc 2580

atctccattt ga 2592

<210> 2
<211> 863
<212> PRT
<213> Lemna paucicostata

<400> 2

Met Ala Gly Phe Leu Gln Lys Val Thr Asp Asn Leu Leu His Lys Ala
1               5                   10                  15

Ala Lys Ile Ser Gly Thr Val Val Leu Val Lys Ser Asn Val Val Gly
            20                  25                  30

Phe Asn Asp Phe Ala Asp Ser Phe Leu Asp Gly Leu His Glu Leu Leu
        35                  40                  45

Gly Ser Gly Ile Thr Phe Gln Leu Val Ser Ala Thr Val Gly Asp Pro

50                  55                  60

Lys Asn Gly Asn Lys Gly Lys Leu Gly Lys Pro Ala Leu Leu Glu Lys
65                  70                  75                  80

Trp Ile Thr Thr Gly Thr Phe Leu Ala Ala Gly Asp Ser Gly Phe Lys
                85                  90                  95

Val Asn Phe Glu Trp Asp Glu Gln Leu Gly Val Pro Gly Ala Val Ile
            100                 105                 110

Val Lys Asn Asn His His Gly Glu Phe Phe Leu Lys Ser Leu Thr Leu
        115                 120                 125

Asp Gly Ala Pro Gly Pro Arg Ser Arg Ile His Phe Asp Cys Asn Ser
    130                 135                 140

Trp Val Tyr Pro Tyr Ser Thr Tyr Asn Tyr Asp Arg Val Phe Phe Ala
145                 150                 155                 160

Asn Asp Thr Tyr Leu Pro Gly Glu Met Pro Glu Pro Leu Lys Ala Tyr
                165                 170                 175

Arg Ala Ala Glu Leu Val Asn Leu Arg Gly Asp Gly Val Thr Arg Glu
            180                 185                 190

Leu Lys Glu Gly Asp Arg Ile Tyr Ala Tyr Asp Leu Tyr Asn Asp Leu
        195                 200                 205

Gly Asp Pro Asp Ser Gly Lys Glu Leu Ala Arg Pro Ile Leu Gly Gly
    210                 215                 220

Ser Glu Glu Tyr Pro Tyr Pro Arg Arg Ala Arg Thr Gly Arg Lys Leu
225                 230                 235                 240

Thr Lys Thr Asp Pro Lys Ser Glu Gln Arg Leu Pro Leu Val Phe Ser
                245                 250                 255

Leu Asn Val Tyr Val Pro Arg Asp Glu Arg Phe Gly His Leu Lys Met
            260                 265                 270

Ser Asp Phe Leu Ala Tyr Ser Leu Lys Ala Leu Ala Gln Gly Leu Val
        275                 280                 285

Pro Ala Leu Asp Ala Ala Thr Asp Ile Thr Pro Phe Glu Phe Asp Thr
    290                 295                 300

Phe Gln Asp Val Leu Asn Leu Tyr Glu Gly Gly Ile Asn Leu Pro Thr
305                 310                 315                 320

Thr Pro Ala Leu Glu Asn Phe Lys Lys Gln Ile Pro Phe Pro Leu Val
                325                 330                 335

Lys Glu Leu Phe Arg Ser Asp Gly Glu Asn Leu Phe Arg Leu Pro Thr
            340                 345                 350

Pro Ser Val Ile Lys Ala Asp Lys Phe Ala Trp Arg Thr Asp Glu Glu
        355                 360                 365

Phe Gly Arg Glu Met Leu Ala Gly Val Asn Pro Val Cys Ile Arg Leu
    370                 375                 380

Leu Lys Lys Phe Pro Pro Val Ser Ser Leu Asp Pro Ser Ile Tyr Arg
385                 390                 395                 400

Asn Gln Asn Ser Thr Met Ile Ala Asp Gln Leu Glu Lys Asn Met Asn
                405                 410                 415

Gly Leu Ser Val Glu Glu Ala Leu Lys Gln Lys Lys Leu Phe Ile Leu
            420                 425                 430

Asp His His Asp Ser Leu Met Pro Tyr Leu Glu Arg Ile Asn Thr Phe
        435                 440                 445

Asn Lys Ile Tyr Ala Ser Arg Thr Val Leu Leu Leu Gln Asp Asp Gly
    450                 455                 460

Thr Leu Lys Pro Leu Ala Ile Glu Leu Ser Leu Pro Val Lys Gly Glu
465                 470                 475                 480

Lys Gly Ala Val Ser Lys Val Tyr Thr Pro Ala Glu His Gly Val Glu
                485                 490                 495

Gly Ala Val Trp Gln Leu Ala Lys Ala Tyr Val Ala Val Asn Asp Ser
            500                 505                 510

Gly Val His Gln Leu Ile Ser His Trp Leu Asn Thr His Ala Thr Ile
        515                 520                 525

Glu Pro Phe Ser Ile Ala Leu Asn Arg Gln Leu Ser Val Val His Pro
    530                 535                 540

Ile Tyr Lys Leu Leu His Pro His Phe Arg Asp Thr Met Asn Ile Asn
545                 550                 555                 560

Ala Phe Ala Arg Gln Ile Leu Ile Asn Ala Gly Gly Ile Leu Glu Met
                565                 570                 575

Thr Val Phe Pro Gly Lys Tyr Ala Met Glu Met Ser Ser Val Val Tyr
            580                 585                 590

Lys Gly Trp Lys Leu Thr Asp Gln Ala Leu Pro Val Asp Leu Leu Asn
        595                 600                 605

Arg Gly Val Ala Glu Lys Asp Pro Ser Ser Gly Glu Leu Arg Leu Leu
    610                 615                 620

Ile Glu Asp Tyr Pro Tyr Ala Val Asp Gly Leu Asp Val Trp Asn Ala
625                 630                 635                 640

Ile Glu Glu Trp Val Lys Glu Tyr Cys Ala Ile Tyr Tyr Pro Ser Asp
                645                 650                 655

Lys Thr Leu Gln Asp Asp Thr Glu Val Gln Ala Trp Trp Lys Glu Val
            660                 665                 670

Arg Glu Val Gly His Gly Asp Lys Lys Asp Glu Thr Trp Trp Pro Ala
        675                 680                 685

Met Glu Thr Val Ala Glu Leu Thr Gln Thr Cys Ser Thr Ile Ile Trp
    690                 695                 700

Val Ala Ser Ala Leu His Ala Ala Val Asn Phe Ala Gln Tyr Pro Tyr
705                 710                 715                 720

Ala Gly Tyr Leu Pro Asn Arg Pro Thr Ile Ser Arg Arg Phe Met Pro
                725                 730                 735

Glu Pro Gly Thr Pro Glu Tyr Glu Glu Leu Glu Thr Asp Pro Asp Arg
            740                 745                 750

Ala Phe Leu Lys Thr Ile Thr Ser Gln Leu Gln Thr Leu Ile Gly Val
        755                 760                 765

Ser Leu Ile Glu Ile Leu Ser Arg His Ser Ser Asp Glu Ile Tyr Leu
    770                 775                 780

Gly Gln Arg Val Ser Ala Glu Trp Thr Ser Asp Ala Lys Ala Leu Ala
785                 790                 795                 800

Ala Phe Lys Ala Phe Gly Glu Lys Leu Ile Thr Ile Glu Asn Lys Ile
                805                 810                 815

Ile Glu Met Asn Val Asp Ser Ser Leu Lys Asn Arg Asn Gly Pro Val
            820                 825                 830

Lys Val Pro Tyr Thr Phe Leu Tyr Pro Asn Thr Thr Asp Tyr Thr Arg
        835                 840                 845

Val Gly Gly Leu Thr Gly Arg Gly Ile Pro Asn Ser Ile Ser Ile
    850                 855                 860

<210>  3
<211>  2610
<212>  DNA
<213>  Lemna paucicostata

<400>  3
atggatgctt ttatccacaa ggtaactgat gtttttacgc acaaggagga gaacaaggtc      60

gccaagatct ccgggactgt ggtgatagtg aagagcaacg tcgtcgggtt caacgacttc     120

gttgactcgt tcctcgacgg cctgcacgag ctcgtcggca gcggcatcac cttccagcta     180

atcagcgcca ccgtcggcga tccaaagaat gggaataaag gcaagctagg aaagccggcg     240

cagttggaga gatggatcac gactgggacc tttctcgctg ctggagactc gggtttcaaa     300

ttgaccttcg agtgggatga gcagctcggc tttcccggag ccgtcatcgt gaaaaacaac     360

caccacggcg agttcttcct caaaagcttc actctggacg gcgtccctgg caagaaaacc     420

cgcattcact tcgactgtaa ctcttgggtc tatccttata gcacctacaa ctacgatcgg     480

gtcttcttcg cgaacgacac attcctaccc ggaaacatgc cggagccgct gaaagcctac     540

agggcggcgg agctggcaaa tctgagaggc gacggagtag ccccccgcga gttgaaggaa     600

ggagatcgaa tttacgctta cgacgtctac aacgacctcg gcgatccgga cagcggcgag     660

gagttggtcc ggccgattct cggcggctcg aaagagtatc cctaccctcg aagagcccgg     720

actggtcgga agatgaccaa gacagatccc aagtcggaga cgaggcttcc acttttgttc     780

agtttgaacg tgtatgtccc cagagacgag cgctttgggc atctgaaaat gtccgatttc     840

ttgggctact cgctcaaggc tctggctcag gggctggtgc ccgtgttaaa tgaggcggcc     900

gacttaactc ctttcgagtt tgatacattc caggacgtat tgaatctcta cgaggggggc     960

atagatctgc ccgacagtcc cgccttagaa aacgtcagga aacagatccc ctttgcactc    1020

ataaaggagc ttttcaggtc cgacggcgag aacctgtttc gccttccgac tcccgccgtt    1080

atcaaagctg acaagttcgc ctggaggact gatgaggagt tcggaaggga aatgcttgcc    1140

ggggtcaacc ctgtctgcat tagactctta acgaaatttc ccccggtcag cagccttgac    1200

cctaacactt acggcgacca caacagtacc atgaccgctg atcagctcga gaagaacatg    1260

aacgggctct ccgtcgaaga ggcccttaat aaaaagaagc tcttcatctt ggaccatcac    1320

gacagtctga tgccctacct agagcgcata aacaccttta acaagatcta cgcctccaga    1380

accgtgttac tgcttcagga cgatggaacc ctaaaaccgc tagccattga gctgagctta    1440

cccatcaagg gtcagaaggg agccgtcagc aaggtataca cgcctgcgga gcatggcgtc    1500

gaggctgccg tgtggcagct cgcgaaggcc ttcgctgcgg tgaacgattc cggcgttcac    1560

cagcttatca gtcactggct gaacacccac gccaccattg agcctttcgc catcgctctc 1620

aacaggcagc tcagcgtagt gcacccaata tacaagctcc ttcatcccca cttccgcgac 1680

accatgaaca tcaacgcctt cgccagacag atcctcatta acgccggcgg aatcctggaa 1740

atgacggtct tcccaggcaa gtatgccttg gagatgtcct ccgtagtgta caaaggctgg 1800

aaactcatcg accacgctct tcccgttgac ctcctcaaca ggggtgtggc tgaaaatgat 1860

ccatccgccc ccggaaagct ccgtttgttg atcgaggact acccgtacgc ggtcgacggg 1920

cttgctgtct ggtctgccat tgagatctgg gtgaaggagt actgcgcaat ctactacccc 1980

tcggacaaga tcctgcagga tgacaccgag ctccaagcct ggtggaagga ggtgcgggag 2040

gtgggacacg gcgacaagaa ggacgaaacg tggtggccga ccatgaagac cgtggccgag 2100

ctgacccaaa cttgctgcac gatcatctgg gtggcctcgg ccctccacgc cgccgtcaac 2160

tttgggcagt acccgtacgc cggctacctc cccaaccgtc ccaccgtaag ccggagattc 2220

atgcccgaac ctggcacgcc tgagtacgaa gagctgaaaa ccgaccccga cagggcgttc 2280

ctcaaaacga ttacaagcca gctgcagacc acaattggcg tgtctctcat cgagatcctc 2340

tcaaggcact cttccgacga ggtctatctc ggtcagagaa tcagcgccga gtggactacg 2400

gacgagaagg cgctggccgc cttcaaggcg tttggtgaga gactgatcac gatcgagaac 2460

cagatcatcg agatgaacca tgagcctaaa ctgaagaacc gtaacggccc cgtgaagctt 2520

ccgtacacac tgctttaccc caacacatcg gattacaccc gcgtgggtgg gctcaccggt 2580

cgcggcatcc ccaatagcgt ctccatttga 2610

<210> 4
<211> 869
<212> PRT
<213> Lemna paucicostata

<400> 4

Met Asp Ala Phe Ile His Lys Val Thr Asp Val Phe Thr His Lys Glu
1               5                   10                  15

Glu Asn Lys Val Ala Lys Ile Ser Gly Thr Val Val Ile Val Lys Ser
            20                  25                  30

Asn Val Val Gly Phe Asn Asp Phe Val Asp Ser Phe Leu Asp Gly Leu
        35                  40                  45

His Glu Leu Val Gly Ser Gly Ile Thr Phe Gln Leu Ile Ser Ala Thr
    50                  55                  60

Val Gly Asp Pro Lys Asn Gly Asn Lys Gly Lys Leu Gly Lys Pro Ala
65                  70                  75                  80

Gln Leu Glu Arg Trp Ile Thr Thr Gly Thr Phe Leu Ala Ala Gly Asp
                85                  90                  95

Ser Gly Phe Lys Leu Thr Phe Glu Trp Asp Glu Gln Leu Gly Phe Pro
            100                 105                 110

Gly Ala Val Ile Val Lys Asn Asn His His Gly Glu Phe Phe Leu Lys
        115                 120                 125

Ser Phe Thr Leu Asp Gly Val Pro Gly Lys Lys Thr Arg Ile His Phe
    130                 135                 140

Asp Cys Asn Ser Trp Val Tyr Pro Tyr Ser Thr Tyr Asn Tyr Asp Arg
145                 150                 155                 160

Val Phe Phe Ala Asn Asp Thr Phe Leu Pro Gly Asn Met Pro Glu Pro
                165                 170                 175

Leu Lys Ala Tyr Arg Ala Ala Glu Leu Ala Asn Leu Arg Gly Asp Gly
            180                 185                 190

Val Ala Pro Arg Glu Leu Lys Glu Gly Asp Arg Ile Tyr Ala Tyr Asp
        195                 200                 205

Val Tyr Asn Asp Leu Gly Asp Pro Asp Ser Gly Glu Glu Leu Val Arg
    210                 215                 220

Pro Ile Leu Gly Gly Ser Lys Glu Tyr Pro Tyr Pro Arg Arg Ala Arg
225                 230                 235                 240

Thr Gly Arg Lys Met Thr Lys Thr Asp Pro Lys Ser Glu Thr Arg Leu
                245                 250                 255

Pro Leu Leu Phe Ser Leu Asn Val Tyr Val Pro Arg Asp Glu Arg Phe
            260                 265                 270

Gly His Leu Lys Met Ser Asp Phe Leu Gly Tyr Ser Leu Lys Ala Leu
        275                 280                 285

Ala Gln Gly Leu Val Pro Val Leu Asn Glu Ala Ala Asp Leu Thr Pro
    290                 295                 300

Phe Glu Phe Asp Thr Phe Gln Asp Val Leu Asn Leu Tyr Glu Gly Gly
305                 310                 315                 320

Ile Asp Leu Pro Asp Ser Pro Ala Leu Glu Asn Val Arg Lys Gln Ile
                325                 330                 335

Pro Phe Ala Leu Ile Lys Glu Leu Phe Arg Ser Asp Gly Glu Asn Leu

340                 345                 350

Phe Arg Leu Pro Thr Pro Ala Val Ile Lys Ala Asp Lys Phe Ala Trp
        355                 360                 365

Arg Thr Asp Glu Glu Phe Gly Arg Glu Met Leu Ala Gly Val Asn Pro
    370                 375                 380

Val Cys Ile Arg Leu Leu Thr Lys Phe Pro Pro Val Ser Ser Leu Asp
385                 390                 395                 400

Pro Asn Thr Tyr Gly Asp His Asn Ser Thr Met Thr Ala Asp Gln Leu
                405                 410                 415

Glu Lys Asn Met Asn Gly Leu Ser Val Glu Glu Ala Leu Asn Lys Lys
            420                 425                 430

Lys Leu Phe Ile Leu Asp His His Asp Ser Leu Met Pro Tyr Leu Glu
        435                 440                 445

Arg Ile Asn Thr Phe Asn Lys Ile Tyr Ala Ser Arg Thr Val Leu Leu
    450                 455                 460

Leu Gln Asp Asp Gly Thr Leu Lys Pro Leu Ala Ile Glu Leu Ser Leu
465                 470                 475                 480

Pro Ile Lys Gly Gln Lys Gly Ala Val Ser Lys Val Tyr Thr Pro Ala
                485                 490                 495

Glu His Gly Val Glu Ala Ala Val Trp Gln Leu Ala Lys Ala Phe Ala
            500                 505                 510

Ala Val Asn Asp Ser Gly Val His Gln Leu Ile Ser His Trp Leu Asn
        515                 520                 525

Thr His Ala Thr Ile Glu Pro Phe Ala Ile Ala Leu Asn Arg Gln Leu
    530                 535                 540

Ser Val Val His Pro Ile Tyr Lys Leu Leu His Pro His Phe Arg Asp
545                 550                 555                 560

Thr Met Asn Ile Asn Ala Phe Ala Arg Gln Ile Leu Ile Asn Ala Gly
                565                 570                 575

Gly Ile Leu Glu Met Thr Val Phe Pro Gly Lys Tyr Ala Leu Glu Met
            580                 585                 590

Ser Ser Val Val Tyr Lys Gly Trp Lys Leu Ile Asp His Ala Leu Pro
        595                 600                 605

Val Asp Leu Leu Asn Arg Gly Val Ala Glu Asn Asp Pro Ser Ala Pro
    610                 615                 620

Gly Lys Leu Arg Leu Leu Ile Glu Asp Tyr Pro Tyr Ala Val Asp Gly
625                 630                 635                 640

Leu Ala Val Trp Ser Ala Ile Glu Ile Trp Val Lys Glu Tyr Cys Ala
                645                 650                 655

Ile Tyr Tyr Pro Ser Asp Lys Ile Leu Gln Asp Asp Thr Glu Leu Gln
            660                 665                 670

Ala Trp Trp Lys Glu Val Arg Glu Val Gly His Gly Asp Lys Lys Asp
        675                 680                 685

Glu Thr Trp Trp Pro Thr Met Lys Thr Val Ala Glu Leu Thr Gln Thr
    690                 695                 700

Cys Cys Thr Ile Ile Trp Val Ala Ser Ala Leu His Ala Ala Val Asn
705                 710                 715                 720

Phe Gly Gln Tyr Pro Tyr Ala Gly Tyr Leu Pro Asn Arg Pro Thr Val
                725                 730                 735

Ser Arg Arg Phe Met Pro Glu Pro Gly Thr Pro Glu Tyr Glu Glu Leu
            740                 745                 750

Lys Thr Asp Pro Asp Arg Ala Phe Leu Lys Thr Ile Thr Ser Gln Leu
        755                 760                 765

Gln Thr Thr Ile Gly Val Ser Leu Ile Glu Ile Leu Ser Arg His Ser
    770                 775                 780

Ser Asp Glu Val Tyr Leu Gly Gln Arg Ile Ser Ala Glu Trp Thr Thr
785                 790                 795                 800

Asp Glu Lys Ala Leu Ala Ala Phe Lys Ala Phe Gly Glu Arg Leu Ile
                805                 810                 815

Thr Ile Glu Asn Gln Ile Ile Glu Met Asn His Glu Pro Lys Leu Lys
            820                 825                 830

Asn Arg Asn Gly Pro Val Lys Leu Pro Tyr Thr Leu Leu Tyr Pro Asn
        835                 840                 845

Thr Ser Asp Tyr Thr Arg Val Gly Gly Leu Thr Gly Arg Gly Ile Pro
    850                 855                 860

Asn Ser Val Ser Ile
865

<210> 5
<211> 2592
<212> DNA
<213> Lemna paucicostata

<400> 5
atggccgatt ttctccaaaa ggtaaccaat aatcttttac acaaggcggc caagatctct 60

gggactgttg tgttagtaaa gagcaatgtc atcgggttca acgatttcgg tgactcgttc 120

ttggacggtc tgcacgaact tctgggcagc ggcatcacct tccagctcgt cagcgccacc 180

gtcggcgatc caaataatgg gaacaagggc aagctaggaa agccggccct gcttgagaaa 240

tggatcacga ccgggacctt tctcgtggcg ggagactctg gcttcaaggt gaacttcgag 300

tgggacgagc agctcgggat tcccggcgca gtcatcgtga aaaacaacca ccacggcgag 360

ttcttcctca agagcttcac tctggatggc gtccctggcc ccagaagccg catccacttc 420

gattgcaact cctgggttta tccctatagc acctacaact acgatagggt tttcttcgct 480

aacgacacgt accttccggg agaaacgccg gagccgctga aagcctacag ggccgcggag 540

ctggtgaatc tgagaggtga cggggtgacc cgcgagctga aggagggaga tcgaatctac 600

gcttacgact actacaacga cctcggcgat ccggacagcg gcaaagagtt agcccggcca 660

atcctcggcg gctcgaaaga gtatccctac cctcgccggg cccggacagg ccggaagttg 720

acgaagactg atccaaagtc ggagcaaagg cttccgctgg tgtttagcct gaacgtgtac 780

gtccccagag acgagcgctt tgggcatctg aagatgtccg atttcttggc ctactccctc 840

aaggctctgg ctcaggggtt ggtgccggcg ttagatgctg cggccgacat aactcctttc 900

gagttcgata ccttccaaga cgtcttgaat ctctacgagg gaggcatcaa tttgcccact 960

actcccgctt cagaaaactt caagaaacag atcccattcc ccctcgtaaa ggagcttttc 1020

aggtccgacg gcgagaacgt gtttcgcctt ccgactccct ccgttatcaa agctgacaag 1080

ttcgcctgga ggactgacga ggagttcgga agggagatgc ttgccggagt caaccccgtt 1140

tgcattagac tcttaacgaa atttccccca gtcagcagtc ttgacccgag catttacggc 1200

aaccagaaca gcaccatgat tgctgatcag ctcgagaaga acatgaacgg gctctccgtc 1260

gaagaggcct taaagcagaa gaagctcttc atcttggacc atcacgatag tctgatgccc 1320

tacctggagc gcataaacac ctttaacaaa atctacgcct ccagaaccgt gttactgctt 1380

cgggacgatg gaaccctaaa accgctagcc attgagctta gcttacccat caaaggcgag 1440

aaaggagctg tcagcaaggt atacacgccg gcagagcatg gcgtcgaggg cgccgtctgg 1500

cagctcgcca aggcctacgt tgccgtcaat gattccggcg ttcaccaact tataagccac 1560

tggctgaaca cccacgccac tatagagcct ttctccatcg ctctcaacag gcagctcagc 1620

gtggtgcacc caatatacaa gctcctccat ccccacttcc gtgacaccat gaacatcaac 1680

gccttcgcca gacaaatcct tattaatgcc ggcggaatcc tcgagatgac ggtcttccca 1740

ggcaagtacg ccatggagat gtcctccgtg gtgtacaaag gatggaaact cacggaccaa 1800

gccctaccgg tggacctcct caacaggggt gtggctgaaa aagatccatc ctcgggagag 1860

cttcggcttt tgatcgagga ctatccgtac gcggtcgacg ggctagacgt ctggaatgcc 1920

attgaggaat gggtcaagga gtactgcgca atctactacc cctcggacaa gaccctgctg 1980

gatgacaccg agctccaagc ctggtggaag gaggtgcgtg aggtgggaca cggtgacaag 2040

aaggatgaaa catggtggcc ggccatggag accgtggccg agctgaccca gacttgctgc 2100

acgatcatct gggtggcctc cgccctccac gccgccgtca actttgcgca gtacccatac 2160

gccggttacc tgcccaaccg tccgactatt agtcgacggt tcatgccaga accgggcacg 2220

ccggagtacg aggagctaga aaccgacccc gaccgggcat tcctcaaaac gattaccagt 2280

cagctgcaga cccttatcgg tgtttctctg atcgagatcc tctccagaca ctcgtctgat 2340

gaaatctatc tagggcagag agttagcgcc gagtggactt cggacgccaa ggcgctggct 2400

gccttcaagg ctttcggtga aaagctgatc accatcgaaa acaagatcat ccagatgaac 2460

gtggactcca gcctgaagaa tcgcaacggc cccgtcaagg tcccgtacac ttttctctac 2520

cccaacacca ccgattatac ccgtgtgggt gggctgaccg gtcgtggaat ccccaacagc 2580

atctccattt ga 2592

<210> 6
<211> 863
<212> PRT
<213> Lemna paucicostata

<400> 6

Met Ala Asp Phe Leu Gln Lys Val Thr Asn Asn Leu Leu His Lys Ala
1               5                   10                  15

Ala Lys Ile Ser Gly Thr Val Val Leu Val Lys Ser Asn Val Ile Gly
            20                  25                  30

Phe Asn Asp Phe Gly Asp Ser Phe Leu Asp Gly Leu His Glu Leu Leu
        35                  40                  45

Gly Ser Gly Ile Thr Phe Gln Leu Val Ser Ala Thr Val Gly Asp Pro
    50                  55                  60

Asn Asn Gly Asn Lys Gly Lys Leu Gly Lys Pro Ala Leu Leu Glu Lys
65                  70                  75                  80

Trp Ile Thr Thr Gly Thr Phe Leu Val Ala Gly Asp Ser Gly Phe Lys
                85                  90                  95

Val Asn Phe Glu Trp Asp Glu Gln Leu Gly Ile Pro Gly Ala Val Ile
            100                 105                 110

Val Lys Asn Asn His His Gly Glu Phe Phe Leu Lys Ser Phe Thr Leu
        115                 120                 125

Asp Gly Val Pro Gly Pro Arg Ser Arg Ile His Phe Asp Cys Asn Ser
    130                 135                 140

Trp Val Tyr Pro Tyr Ser Thr Tyr Asn Tyr Asp Arg Val Phe Phe Ala
145                 150                 155                 160

Asn Asp Thr Tyr Leu Pro Gly Glu Thr Pro Glu Pro Leu Lys Ala Tyr
                165                 170                 175

Arg Ala Ala Glu Leu Val Asn Leu Arg Gly Asp Gly Val Thr Arg Glu
            180                 185                 190

Leu Lys Glu Gly Asp Arg Ile Tyr Ala Tyr Asp Tyr Tyr Asn Asp Leu
        195                 200                 205

Gly Asp Pro Asp Ser Gly Lys Glu Leu Ala Arg Pro Ile Leu Gly Gly
    210                 215                 220

Ser Lys Glu Tyr Pro Tyr Pro Arg Arg Ala Arg Thr Gly Arg Lys Leu
225                 230                 235                 240

Thr Lys Thr Asp Pro Lys Ser Glu Gln Arg Leu Pro Leu Val Phe Ser
                245                 250                 255

Leu Asn Val Tyr Val Pro Arg Asp Glu Arg Phe Gly His Leu Lys Met
            260                 265                 270

Ser Asp Phe Leu Ala Tyr Ser Leu Lys Ala Leu Ala Gln Gly Leu Val
        275                 280                 285

Pro Ala Leu Asp Ala Ala Ala Asp Ile Thr Pro Phe Glu Phe Asp Thr
    290                 295                 300

Phe Gln Asp Val Leu Asn Leu Tyr Glu Gly Gly Ile Asn Leu Pro Thr
305                 310                 315                 320

Thr Pro Ala Ser Glu Asn Phe Lys Lys Gln Ile Pro Phe Pro Leu Val
                325                 330                 335

Lys Glu Leu Phe Arg Ser Asp Gly Glu Asn Val Phe Arg Leu Pro Thr
            340                 345                 350

Pro Ser Val Ile Lys Ala Asp Lys Phe Ala Trp Arg Thr Asp Glu Glu
        355                 360                 365

Phe Gly Arg Glu Met Leu Ala Gly Val Asn Pro Val Cys Ile Arg Leu
    370                 375                 380

Leu Thr Lys Phe Pro Pro Val Ser Ser Leu Asp Pro Ser Ile Tyr Gly
385                 390                 395                 400

Asn Gln Asn Ser Thr Met Ile Ala Asp Gln Leu Glu Lys Asn Met Asn
                405                 410                 415

Gly Leu Ser Val Glu Glu Ala Leu Lys Gln Lys Lys Leu Phe Ile Leu
            420                 425                 430

Asp His His Asp Ser Leu Met Pro Tyr Leu Glu Arg Ile Asn Thr Phe
        435                 440                 445

Asn Lys Ile Tyr Ala Ser Arg Thr Val Leu Leu Leu Arg Asp Asp Gly
    450                 455                 460

Thr Leu Lys Pro Leu Ala Ile Glu Leu Ser Leu Pro Ile Lys Gly Glu
465                 470                 475                 480

Lys Gly Ala Val Ser Lys Val Tyr Thr Pro Ala Glu His Gly Val Glu
                485                 490                 495

Gly Ala Val Trp Gln Leu Ala Lys Ala Tyr Val Ala Val Asn Asp Ser
            500                 505                 510

Gly Val His Gln Leu Ile Ser His Trp Leu Asn Thr His Ala Thr Ile
        515                 520                 525

Glu Pro Phe Ser Ile Ala Leu Asn Arg Gln Leu Ser Val Val His Pro
    530                 535                 540

Ile Tyr Lys Leu Leu His Pro His Phe Arg Asp Thr Met Asn Ile Asn
545                 550                 555                 560

Ala Phe Ala Arg Gln Ile Leu Ile Asn Ala Gly Gly Ile Leu Glu Met
                565                 570                 575

Thr Val Phe Pro Gly Lys Tyr Ala Met Glu Met Ser Ser Val Val Tyr
            580                 585                 590

Lys Gly Trp Lys Leu Thr Asp Gln Ala Leu Pro Val Asp Leu Leu Asn
        595                 600                 605

Arg Gly Val Ala Glu Lys Asp Pro Ser Ser Gly Glu Leu Arg Leu Leu

610                 615                 620

Ile Glu Asp Tyr Pro Tyr Ala Val Asp Gly Leu Asp Val Trp Asn Ala
625                 630                 635                 640

Ile Glu Glu Trp Val Lys Glu Tyr Cys Ala Ile Tyr Tyr Pro Ser Asp
                645                 650                 655

Lys Thr Leu Leu Asp Asp Thr Glu Leu Gln Ala Trp Trp Lys Glu Val
            660                 665                 670

Arg Glu Val Gly His Gly Asp Lys Lys Asp Glu Thr Trp Trp Pro Ala
        675                 680                 685

Met Glu Thr Val Ala Glu Leu Thr Gln Thr Cys Cys Thr Ile Ile Trp
    690                 695                 700

Val Ala Ser Ala Leu His Ala Ala Val Asn Phe Ala Gln Tyr Pro Tyr
705                 710                 715                 720

Ala Gly Tyr Leu Pro Asn Arg Pro Thr Ile Ser Arg Arg Phe Met Pro
                725                 730                 735

Glu Pro Gly Thr Pro Glu Tyr Glu Glu Leu Glu Thr Asp Pro Asp Arg
            740                 745                 750

Ala Phe Leu Lys Thr Ile Thr Ser Gln Leu Gln Thr Leu Ile Gly Val
        755                 760                 765

Ser Leu Ile Glu Ile Leu Ser Arg His Ser Ser Asp Glu Ile Tyr Leu
    770                 775                 780

Gly Gln Arg Val Ser Ala Glu Trp Thr Ser Asp Ala Lys Ala Leu Ala
785                 790                 795                 800

Ala Phe Lys Ala Phe Gly Glu Lys Leu Ile Thr Ile Glu Asn Lys Ile
                805                 810                 815

Ile Gln Met Asn Val Asp Ser Ser Leu Lys Asn Arg Asn Gly Pro Val
            820                 825                 830

Lys Val Pro Tyr Thr Phe Leu Tyr Pro Asn Thr Thr Asp Tyr Thr Arg
        835                 840                 845

Val Gly Gly Leu Thr Gly Arg Gly Ile Pro Asn Ser Ile Ser Ile
    850                 855                 860

<210>  7
<211>  24

<212>  DNA
<213>  Artifical Sequence

<220>
<221>  misc_feature
<222>  (3)..(12)
<223>  n is inosine


<400>  7
gcntggmgna cngaygarga rtty                                              24


<210>  8
<211>  23
<212>  DNA
<213>  Artifical Sequence

<220>
<221>  misc_feature
<222>  (5)..(15)
<223>  n is inosine


<400>  8
gcrtanggrt aytgnccraa rtt                                               23


<210>  9
<211>  18
<212>  DNA
<213>  Artifical Sequence

<400>  9
agctcttcat cttggacc                                                     18


<210>  10
<211>  22
<212>  DNA
<213>  Artifical Sequence

<400>  10
aagcttcttc atccccactt cc                                                22


<210>  11
<211>  22
<212>  DNA
<213>  Artifical Sequence

<400>  11
aagctccttc atccccactt cc                                                22


<210>  12
<211>  18
<212>  DNA
<213>  Artifical Sequence

<400>  12
tttcatcctt cttgtcgc                                                     18


<210>  13

<211>  19
<212>  DNA
<213>  Artifical Sequence

<400>  13
gcttgtatat tgggtgcac                                                    19


<210>  14
<211>  23
<212>  DNA
<213>  Artifical Sequence

<400>  14
aagcagtaac acggttctgg agg                                               23